(19) **Europäisches Patentamt / European Patent Office / Office européen des brevets**

(11) **EP 3 284 781 B1**

(12) **EUROPEAN PATENT SPECIFICATION**

(45) Date of publication and mention
of the grant of the patent:
**28.10.2020 Bulletin 2020/44**

(21) Application number: **16779790.1**

(22) Date of filing: **18.01.2016**

(51) Int Cl.:
*C08L 11/02* (2006.01)     *A41D 19/00* (2006.01)
*A41D 19/015* (2006.01)     *A41D 19/04* (2006.01)
*B29C 41/14* (2006.01)     *C08K 3/22* (2006.01)
*C08K 5/00* (2006.01)     *B29K 21/00* (2006.01)
*B29L 31/48* (2006.01)     *C08J 5/02* (2006.01)
*C08K 5/13* (2006.01)

(86) International application number:
**PCT/JP2016/051257**

(87) International publication number:
**WO 2016/166998 (20.10.2016 Gazette 2016/42)**

(54) **COMPOSITION FOR RUBBER AND USE THEREOF**

ZUSAMMENSETZUNG FÜR GUMMI UND VERWENDUNG DAVON

COMPOSITION POUR CAOUTCHOUC ET SON UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.04.2015   JP 2015084274**

(43) Date of publication of application:
**21.02.2018   Bulletin 2018/08**

(73) Proprietor: **Showa Denko K.K.
Tokyo 105-8518 (JP)**

(72) Inventors:
• **OGAWA Noriko
Tokyo 105-8518 (JP)**

• **TAKENOSHITA Youichiro
Tokyo 105-8518 (JP)**

(74) Representative: **Strehl Schübel-Hopf & Partner
Maximilianstrasse 54
80538 München (DE)**

(56) References cited:
**WO-A1-2012/137663     WO-A1-2013/015043
WO-A1-2013/129676     JP-A- 2006 219 546
JP-A- 2007 106 994     JP-A- 2011 122 141
JP-A- 2013 508 528**

**Description**

Technical Field

The present invention relates to a composition for rubber containing a chloroprene-based polymer latex and a molded article obtained using the same. More specifically, the present invention relates to a composition for rubber containing a chloroprene-based polymer latex having a specific structure, a metal oxide, an antioxidant, a surfactant, and a pH adjuster, and being free of a vulcanization accelerator. The molded article obtained from the composition for rubber of the present invention containing the chloroprene-based polymer latex is suitably used in, for example, dipped articles, such as gloves, a sphygmomanometer bladder, and rubber thread, in particular, a medical glove application.

Background Art

Hitherto, a material using a chloroprene-based polymer latex, which is satisfactory in terms of characteristics such as general rubber physical properties, weatherability, heat resistance, and chemical resistance, has been widely used in: dipping applications, such as gloves; pressure-sensitive adhesive/adhesive applications; civil engineering and architectural applications, such as elastic asphalt (modified asphalt) and elastic cement; and the like. In disposable medical glove applications, in particular, surgical gloves, a shock symptom (anaphylaxis) due to an allergy to natural rubber is a serious problem in terms of hygiene and life safety to patients and medical technicians. In order to solve the problem, a chloroprene rubber (hereinafter sometimes abbreviated as "CR"), which has flexibility and mechanical characteristics similar to those of the natural rubber, and is relatively inexpensive, has been used as a material for the surgical gloves. The chloroprene rubber (CR) specifically has advantages of being excellent in fitting sense (comfort) and response to fine movement of a fingertip (followability), which are similar to those of the natural rubber.

However, the hitherto known chloroprene rubber (CR) is insufficient in terms of structure of a polymer contained in the chloroprene-based polymer latex, and hence use of a vulcanization accelerator has been indispensable for obtaining a vulcanized rubber having target strength. In recent years, there has been a case of development of contact dermatitis by synthetic rubber gloves. Such case is due to the vulcanization accelerator to be used in forming the chloroprene-based polymer latex into a molded article, and is called a type IV allergy. In view of this, there has been an increasing demand for gloves free of the vulcanization accelerator that is an allergen of the type IV allergy.

There is a proposal of a method involving using a chloroprene-based polymer latex for surgical gloves to improve flexibility thereof (for example, JP 2007-106994 A; Patent Document 1, and JP 2009-501833 A (EP 1904569 B1); Patent Document 2).

In the case of Patent Document 1, use of the vulcanization accelerator is essential, and hence the problem of the type IV allergy cannot be solved. In addition, in the case of Patent Document 2, there are problems in that: when a solid content is low, film formation is difficult; and when the solid content is high, a compound is deteriorated in storage stability, and hence is liable to aggregate, resulting in impairment in external appearance of an article. Therefore, there have been desired a chloroprene-based polymer latex capable of providing a vulcanized rubber having excellent mechanical characteristics and a composition containing the same.

Citation List

Patent Document

[Patent Document 1] JP 2007-106994 A
[Patent Document 2] JP 2009-501833 A (EP 1904569 B1)

Summary of Invention

Technical Problem

It is an object of the present invention to provide a composition for rubber containing a chloroprene-based polymer latex capable of being crosslinked to form a chloroprene rubber (CR) suited for applications such as surgical gloves without using a vulcanization accelerator that is a causative substance (allergen) of a type IV allergy.

Solution to Problem

The inventors of the present invention have made extensive investigations in order to achieve the object, and as a result, have found that the above-mentioned problems can be solved with a molded article obtained from a composition for rubber containing a chloroprene-based polymer latex that provides a polymer having a specific structure, and also containing a metal oxide, an antioxidant, a surfactant, and a pH adjuster.

That is, the present invention relates to the following items [1] to [9].

[1] A composition for rubber, comprising a chloroprene-based polymer latex (A), a metal oxide (B), an antioxidant (C), a surfactant (D) and a pH adjuster (E), and being free of a vulcanization accelerator, in which a tetrahydrofuran insoluble matter content in a chloroprene-based polymer contained in the (A) is from 50 mass% to 85 mass%, and in which contents of the (B), the (C), the (D), and the (E) with respect to 100 parts by mass of a solid content of the (A) are from 1 part by mass to 10 parts by mass, from 0.1 part by mass to 5 parts by mass, from 0.1 part by mass to 10 parts by mass, and from 0.01 part by mass to 5 parts by mass, respectively.

[2] The composition for rubber according to [1] above, which is a composition comprising the chloroprene-based polymer latex (A), the metal oxide (B), the antioxidant (C), the surfactant (D), and the pH adjuster (E).

[3] The composition for rubber according to [1] or [2] above, in which a polymer contained in the chloroprene-based polymer latex (A) is a copolymer formed from monomers comprising 2-chloro-1,3-butadiene (chloroprene) (A-1) and 2,3-dichloro-1,3-butadiene (A-2), and in which ratios of the monomers with respect to 100 mass% in total of the 2-chloro-1,3-butadiene (chloroprene) (A-1) and the 2,3-dichloro-1,3-butadiene (A-2) are 76 mass% to 93 mass% of the 2-chloro-1,3-butadiene (chloroprene) (A-1) and 24 mass% to 7 mass% of the 2,3-dichloro-1,3-butadiene (A-2).

[4] The composition for rubber according to [3] above, in which the polymer contained in the chloroprene-based polymer latex (A) is a copolymer formed from the 2-chloro-1,3-butadiene (chloroprene) (A-1) and the 2,3-dichloro-1,3-butadiene (A-2), and further, a monomer (A-3) copolymerizable therewith, and in which a content of the monomer (A-3) is from 0.1 part by mass to 10 parts by mass with respect to 100 parts by mass in total of the 2-chloro-1,3-butadiene (chloroprene) (A-1) and the 2,3-dichloro-1,3-butadiene (A-2).

[5] A molded article, which is obtained using the composition for rubber according to any one of [1] to [4] above.

[6] The molded article according to [5] above, which has a 300% modulus of elasticity of from 0.5 MPa to 1.2 MPa, a tensile strength of 17 MPa or more, a tensile elongation at break of 800% or more, and a deformation ratio of 20% or less.

[7] A dipped rubber article, which is obtained using the composition for rubber according to any one of [1] to [4] above.

[8] The dipped rubber article according to [7] above, in which the dipped rubber article is a glove.

[9] The dipped rubber article according to [8] above, in which the glove is a disposable medical glove.

Advantageous Effects of Invention

The molded article obtained from the composition for rubber of the present invention containing a chloroprene-based polymer latex having a specific structure, a metal oxide, an antioxidant, a surfactant and a pH adjuster, and being free of the vulcanization accelerator is excellent in tensile strength, flexibility, and stability of the flexibility, and can be suitably used as molded articles, for example, dipped articles such as gloves, a sphygmomanometer bladder, and rubber thread, each of which is capable of avoiding the type IV allergy.

Description of Embodiments

A composition for rubber according to the present invention has features of containing a chloroprene-based polymer latex (A) having a specific structure, a metal oxide (B), an antioxidant (C), a surfactant (D), and a pH adjuster (E), and being free of a vulcanization accelerator that is an allergen of a type IV allergy.

As the composition for rubber according to the present invention, a composition formed of a chloroprene-based polymer latex (A), a metal oxide (B), an antioxidant (C), a surfactant (D) and a pH adjuster (E) is preferred.

The chloroprene-based polymer latex constituting the composition for rubber of the present invention contains, as comonomers, 2-chloro-1,3-butadiene (chloroprene) (A-1) and 2,3-dichloro-1,3-butadiene (A-2), and in the chloroprene-based polymer latex, the content of the 2,3-dichloro-1,3-butadiene (A-2) falls within the range of from 7 mass% to 24 mass% with respect to 100 mass% in total of both the comonomers, a polymerization temperature (T) falls within the range of T=from 25°C to 45°C, the mass% of 2,3-dichloro-1,3-butadiene falls within the range of the formula (I) specified in the function of T to be described later, and a tetrahydrofuran insoluble matter content in a chloroprene-based polymer in the chloroprene-based polymer latex (A) is from 50 mass% to 85 mass%.

When the tetrahydrofuran insoluble matter content is set to fall within the above-mentioned range, the lack of mechanical physical properties, such as tensile strength, due to the absence of the vulcanization accelerator can be sufficiently ameliorated.

Emulsion polymerization may be employed for the preparation of the chloroprene-based polymer latex (A) having a specific structure serving as a component of the composition for rubber of the present invention. From an industrial point of view, aqueous emulsion polymerization is preferred. As an emulsifier for the emulsion polymerization, a general rosin acid soap may be used in view of the convenience of a coagulation operation. In particular, a sodium and/or potassium salt of disproportionated rosin acid is preferred from the viewpoint of coloration stability. The use amount of the rosin acid soap is preferably from 3 mass% to 8 mass% with respect to 100 mass% of the monomers. When the use amount is less than 3 mass%, an emulsification failure occurs, and hence problems, such as the deterioration of polymerization heat generation control, the generation of an aggregate, and a failure in external appearance of an article, are liable to occur. A case in which the use amount is more than 8 mass% is not preferred because the polymer is liable to undergo pressure-sensitive adhesion owing to residual rosin acid, and hence processability and handleability are deteriorated owing to pressure-sensitive adhesion to a mold (former) at the time of the molding of a part, pressure-sensitive adhesion at the time of the use of the part, and the like, and in addition, the color tone of the article is deteriorated.

In a method of producing the chloroprene-based polymer latex (A) having a specific structure, the 2,3-dichloro-1,3-butadiene (A-2) is used as a monomer because its copolymerizability with the 2-chloro-1,3-butadiene (chloroprene) (A-1) is satisfactory, and hence crystallization resistance, and furthermore, characteristics such as flexibility can be easily adjusted. The fraction of the 2,3-dichloro-1,3-butadiene (A-2) is, with respect to 100 mass% in total of the 2-chloro-1,3-butadiene (chloroprene) (A-1) and the 2,3-dichloro-1,3-butadiene (A-2), preferably from 7 mass% to 24 mass%, more preferably from 10 mass% to 15 mass%. When the fraction of the A-2 is 7 mass% or more, the improvement of the temporal stability of the flexibility is satisfactory. When the fraction is 24 mass% or less, the crystallization of the polymer is suppressed, and hence the flexibility is satisfactory. For example, any of 1-chloro-1,3-butadiene, butadiene, isoprene, styrene, acrylonitrile, acrylic acid and esters thereof, and methacrylic acid and esters thereof may be used as a monomer (A-3) copolymerizable with the 2-chloro-1,3-butadiene (chloroprene) (A-1) and the 2,3-dichloro-1,3-butadiene (A-2) in the range of from 0.1 part by mass to 10 parts by mass with respect to 100 parts by mass in total of the 2-chloro-1,3-butadiene (chloroprene) (A-1) and the 2,3-dichloro-1,3-butadiene (A-2) as long as the object of the present invention is not inhibited. Two or more kinds thereof may be used as necessary. When the amount of the (A-3) is set to 10 parts by mass or less, the temporal stability of the flexibility as well as tensile strength and elongation can be satisfactorily maintained.

When the polymerization is performed in such a manner that: the polymerization temperature T falls within the range of from 25°C to 45°C; with respect to 100 mass% in total of the 2-chloro-1,3-butadiene (chloroprene) (A-1) and the 2,3-dichloro-1,3-butadiene (A-2), the mass% (M) of the 2,3-dichloro-1,3-butadiene (A-2) satisfies the following expression (I):

$$7-0.44(T-45) \leq M \leq 15-0.44(T-45) \qquad (I);$$

and the polymer has a tetrahydrofuran insoluble matter content of from 50 mass% to 85 mass%, the polymerization rates of the comonomers can be balanced.

A chain transfer agent is not particularly limited, and xanthic disulfide or an alkyl mercaptan may be used. A specific example thereof is n-dodecyl mercaptan.

The polymerization conversion of the chloroprene-based polymer latex (A) having a specific structure is preferably from 80% to 95%. A case in which the polymerization conversion is less than 80% is not preferred because the solid content of the polymer latex is lowered to apply a burden to a drying step or make it difficult to form a film, with the result that a pinhole or a crack is liable to occur. A case in which the polymerization conversion is more than 95% is not preferred because a polymerization time lengthens to deteriorate productivity, and moreover, a problem occurs also in, for example, that the mechanical strength of the film is deteriorated and the film becomes brittle.

The tetrahydrofuran insoluble matter content of the polymer contained in the chloroprene-based polymer latex having a specific structure is preferably from 50 mass% to 85 mass%, more preferably from 60 mass% to 85 mass%. A case in which the tetrahydrofuran insoluble matter content is less than 50 mass% is not preferred because the tensile strength is decreased or pressure-sensitive adhesion to a hand mold at the time of molding is enhanced to make release therefrom difficult. In addition, when the tetrahydrofuran insoluble matter content is more than 85 mass%, the polymer becomes brittle, and the tensile strength and the tensile elongation as well as the flexibility are deteriorated.

The tetrahydrofuran insoluble matter content can be easily controlled to from 50 mass% to 85 mass% by adjusting the amount of the chain transfer agent within a range in which the polymerization conversion of the chloroprene-based polymer latex (A) is from 80% to 95%.

As an initiator for the polymerization, a general radical polymerization initiator may be used. For example, in the case of emulsion polymerization, a general organic or inorganic peroxide such as benzoyl peroxide, potassium persulfate or ammonium persulfate, or an azo compound such as azobisisobutyronitrile is used. In combination therewith, a promoter such as an anthraquinonesulfonic acid salt, potassium sulfite, or sodium sulfite may be used as appropriate.

In general, in the production of a chloroprene-based polymer, a polymerization terminator is added to terminate the reaction at a time point when a predetermined polymerization ratio is reached, for the purpose of obtaining a polymer having a desired molecular weight and distribution. The polymerization terminator is not particularly limited, and for example, generally used terminators, such as phenothiazine, p-t-butylcatechol, hydroquinone, hydroquinone monomethyl ether, and diethylhydroxylamine, may be used.

A chloroprene-based polymer is generally liable to be degraded by oxygen. In the present invention, it is desired that a stabilizer, such as an acid acceptor or an antioxidant, be used as appropriate within a range in which the effects of the invention are not impaired.

When 1 part by mass to 10 parts by mass of the metal oxide (B), 0.1 part by mass to 5 parts by mass of the antioxidant (C), 0.1 part by mass to 10 parts by mass of the surfactant (D), and 0.01 part by mass to 5 parts by mass of the pH adjuster (E) are compounded with respect to 100 parts by mass of the solid content of the chloroprene-based polymer latex (A), a composition for rubber having sufficient tensile strength and flexibility is obtained. Of the raw materials to be used for compounding, one that is insoluble in water, or that destabilizes the colloid state of the polymer latex is added to the polymer latex by producing an aqueous dispersion thereof in advance of the addition.

The metal oxide (B) is not particularly limited, and specific examples thereof include zinc oxide, lead oxide, and trilead tetroxide. In particular, zinc oxide is preferred. Those metal oxides may be used in combination thereof. The addition amount of any such metal oxide is preferably from 1 part by mass to 10 parts by mass with respect to 100 parts by mass of the solid content of the chloroprene-based polymer latex. When the addition amount of the metal oxide is less than 1 part by mass, a crosslinking rate is not sufficient. In contrast, when the addition amount is more than 10 parts by mass, crosslinking becomes so fast that a scorch is liable to occur. In addition, the colloid stability of the composition of the polymer latex is deteriorated, with the result that a problem such as sedimentation is liable to occur.

With regard to the antioxidant (C), when extreme heat resistance is required, an antioxidant intended to impart heat resistance (anti-heat aging agent) and an antioxidant against ozone (anti-ozone aging agent) need to be used, and these antioxidants are preferably used in combination thereof. As the anti-heat aging agent, a diphenylamine-based anti-heat aging agent such as octylated diphenylamine, p-(p-toluene-sulfonylamide)diphenylamine, or 4,4'-bis($\alpha,\alpha$-dimethylbenzyl)diphenylamine is preferably used because such agent has contamination resistance (has less migration of color or the like) as well as heat resistance. As the anti-ozone aging agent, N,N'-diphenyl-p-phenylenediamine (DPPD) or N-isopropyl-N'-phenyl-p-phenylenediamine (IPPD) is used. However, when an external appearance, in particular, a color tone, and hygiene are regarded as important as in medical gloves and the like, a hindered phenol-based antioxidant is generally used. The addition amount of the antioxidant (C) is preferably from 0.1 part by mass to 5 parts by mass with respect to 100 parts by mass of the solid content of the chloroprene-based polymer latex (A). When the addition amount of the antioxidant (C) is less than 0.1 part by mass, an antioxidant effect is not sufficient. In contrast, when the addition amount is more than 5 parts by mass, the crosslinking is inhibited or the color tone is deteriorated.

As the surfactant (D), a sodium alkyl sulfate, a sodium alkylbenzenesulfonate, a sodium naphthalene sulfonate formaldehyde condensate, a rosin acid soap, a fatty acid soap, or the like is used. The addition amount of the surfactant is preferably from 0.1 part by mass to 10 parts by mass with respect to 100 parts by mass of the solid content of the chloroprene-based polymer latex. When the addition amount is less than 0.1 part by mass, colloid stabilization is insufficient. When the addition amount is more than 10 parts by mass, foaming and a defect in external appearance of an article, such as a pinhole, are liable to be caused.

As the pH adjuster (E), an alkali or a weak acid such as an amino acid or acetic acid is used for the purpose of imparting colloid stability or adjusting a film thickness. Examples of the alkali include potassium hydroxide and ammonia, and an example of the weak acid is glycine. The pH adjuster is prepared as an aqueous solution before use so as to be diluted to such a degree that no shock is applied to the colloid stability. The addition amount of the pH adjuster is preferably from 0.01 part by mass to 5 parts by mass with respect to 100 parts by mass of the solid content of the chloroprene-based polymer latex. When the addition amount of the pH adjuster is less than 0.01 part by mass, colloid stabilization and film thickness adjustment become insufficient. In contrast, when the addition amount is more than 5 parts by mass, coagulation becomes insufficient or an aggregate is generated in a compound.

In the present invention, a vulcanization accelerator serving as a cause of a type IV allergy is not used. Therefore, when used as medical gloves, the composition for rubber of the present invention can be safely used without concern for an allergy.

A vulcanization accelerator that has heretofore been generally used for the vulcanization of the chloroprene-based polymer latex is a thiuram-based, dithiocarbamate-based, thiourea-based, or guanidine-based vulcanization accelerator. Examples of the thiuram-based vulcanization accelerator include tetraethylthiuram disulfide and tetrabutylthiuram disulfide. Examples of the dithiocarbamate-based vulcanization accelerator include sodium dibutylthiodicarbamate, zinc dibutylthiodicarbamate, and zinc diethylthiodicarbamate. Examples of the thiourea-based vulcanization accelerator include ethylene thiourea, diethylthiourea, trimethylthiourea, and N,N'-diphenylthiourea. Examples of the guanidine-based vulcanization accelerator include diphenylguanidine and di-o-toluylguanidine. In addition, the vulcanization accelerators given above are used in combination thereof in some cases. However, in the present invention, those vulcanization accelerators are not used at all.

The chloroprene-based polymer having a specific structure obtained by the above-mentioned method is: a copolymer in which fractions of the respective monomers are 76 mass% to 93 mass% of the 2-chloro-1,3-butadiene (chloroprene) (A-1) and 24 mass% to 7 mass% of the 2,3-dichloro-1,3-butadiene (A-2) when the total amount of all monomers is set to 100 mass%; or a copolymer further containing, in addition to the 2-chloro-1,3-butadiene (chloroprene) (A-1) and the 2,3-dichloro-1,3-butadiene (A-2), 0.1 part by mass to 10 parts by mass of the monomer (A-3) copolymerizable therewith with respect to 100 parts by mass in total of the 2-chloro-1,3-butadiene (chloroprene) (A-1) and the 2,3-dichloro-1,3-butadiene (A-2), and the polymer has a tetrahydrofuran insoluble matter content of from 50 mass% to 85 mass%.

The composition of the monomers in the chloroprene-based polymer having a specific structure is not necessarily the same as the composition of the monomers as fed because the consumption amounts of the monomers during the polymerization vary depending on the kinds of the monomers. Therefore, the polymerization conversion influences the composition of the monomers in the polymer to be generated. When the 2-chloro-1,3-butadiene (chloroprene) (A-1) and the 2,3-dichloro-1,3-butadiene (A-2) are copolymerized, the 2,3-dichloro-1,3-butadiene (A-2) is liable to be consumed at the initial stage of the polymerization, and hence the ratio of the 2-chloro-1,3-butadiene (chloroprene) (A-1) serving as the remaining unreacted monomer increases. Thus, in general, the ratio of the 2,3-dichloro-1,3-butadiene (A-2) in the polymer is larger than the ratio of the 2,3-dichloro-1,3-butadiene (A-2) as fed.

The composition for rubber of the present invention comprising a chloroprene-based polymer latex having a specific structure, a metal oxide, an antioxidant, a surfactant, and a pH adjuster is obtained as a film-like rubber composition by a general method proceeding with the steps of dipping and coagulation, leaching (removal of water-soluble impurities), drying, and crosslinking in the stated order. In those steps, in particular, a crosslinking temperature needs attention because high temperature is required for obtaining a desired degree of crosslinking, as compared to the case of natural rubber. For the purpose of avoiding problems with the external appearance of an article, such as a blister and a pinhole, rough drying at a relatively low temperature within the range of from 70°C to 100°C is needed in some cases in advance of the crosslinking. A crosslinking temperature of from 120°C to 140°C and a crosslinking time of from 30 minutes to 2 hours are needed. As an indicator of the degree of crosslinking, a deformation ratio is often used. A lack of crosslinking results in a lack of elasticity of the film, and consequently a deformation ratio when a glove is completely elongated is large, with the result that the glove does not fit a hand sufficiently. Therefore, the deformation ratio is preferably as small as possible. As the degree of crosslinking increases (as the crosslinking comes closer to completion), the deformation ratio reduces. The crosslinking is preferably performed sufficiently within a range in which other physical properties, such as the tensile strength and elongation at break, are not deteriorated. In this case, the deformation ratio is preferably 20% or less, more preferably 15% or less. The film after the crosslinking may be measured for its modulus of elasticity, tensile strength, and tensile elongation at break by being subjected to a tensile test. When the desired degree of crosslinking is achieved, that is, when the deformation ratio is 20% or less, the crosslinked film obtained from the composition comprising a chloroprene-based polymer latex having a specific structure, a metal oxide, an antioxidant, a surfactant, and a pH adjuster can achieve a 300% modulus of elasticity of from 0.5 MPa to 1.2 MPa, a tensile strength of 17 MPa or more, more preferably 20 MPa or more, and a tensile elongation at break of 800% or more.

A rubber produced through the crosslinking under such conditions as described above maintains basic characteristics intrinsic to the chloroprene-based polymer and provides excellent flexibility, while avoiding the type IV allergy due to the vulcanization accelerator.

Examples

The present invention is described below by way of Production Example, Examples, and Comparative Examples, but the present invention is not limited to the following examples.

Polymerization Conversion:

An emulsion after polymerization was collected and dried at 100°C for 2 hours. On the basis of the resultant solid content, a polymerization conversion was calculated.

The solid content and the polymerization conversion were determined by the following equations.

$$\text{Solid content [mass\%]} = [(\text{weight after drying at 100°C for 2 hours})/(\text{latex weight before drying})] \times 100$$

$$\text{Polymerization conversion [\%]} = [(\text{polymer generation amount/monomer feeding amount})] \times 100$$

In this case, the generation amount of a polymer was determined by subtracting the solid content excluding the polymer from the solid content after polymerization.

Physical Properties of Chloroprene-based Polymer Latex:

Physical properties of a chloroprene-based polymer latex were evaluated by the following methods.

[Measurement Methods]

Tetrahydrofuran Insoluble Matter Content:

1 g of the latex was added dropwise to 100 ml of a tetrahydrofuran (THF) solvent, and the mixture was shaken overnight. After that, a dissolved phase in a supernatant was separated with a centrifuge, and the solvent was evaporated to dryness at 100°C over 1 hour. Then, a dissolved matter content was calculated and subtracted to evaluate a tetrahydrofuran insoluble matter content.

Copolymerization Fraction of 2,3-Dichloro-1,3-butadiene:

A residual 2-chloro-1,3-butadiene (chloroprene) monomer and 2,3-dichloro-1,3-butadiene monomer in the emulsion after polymerization were analyzed with a gas chromatograph, and were subtracted from the amounts of the monomers as fed to calculate copolymerization composition in the polymer.

Physical Properties after Crosslinking:

A chloroprene-based polymer latex compound was produced in the compounding ratio shown in Table 1 below.

Table 1

|  | Part(s) by mass |
| --- | --- |
| Chloroprene-based polymer latex | 100 |
| Surfactant (1) | 1 |
| Surfactant (2) | 2 |
| Zinc oxide dispersion (3) | 5 |
| Phenol-based antioxidant dispersion (4) | 2 |
| Glycine | 0.5 |

Notes:
(1) Darvan SMO manufactured by Kawaguchi Chemical Industry, Co., Ltd.
(2) Darvan WAQ manufactured by Kawaguchi Chemical Industry, Co., Ltd.
(3) AZ-SW manufactured by Osaki Industry, Co., Ltd.
(4) K-840 (Wingstay (trademark) L dispersion) manufactured by Chukyo Yushi Co., Ltd.

Mixing to Homogeneity:

The compound was fed into a stirring vessel with a three-one motor, and stirred for 30 minutes.

Production of Polymer Film:

A dipped film was produced from the composition for rubber, which had been obtained by compounding the chloroprene-based polymer latex and the like, by the following method.

Coagulation, Leaching, and Drying:

A 25% aqueous solution of calcium nitrate was used as a coagulation liquid to provide a dipped film. After that, leaching was performed in warm water at 70°C for 2 minutes to remove water-soluble components. Then, drying was performed at 70°C for 30 minutes.

Crosslinking:

Crosslinking was performed by heating in an oven in accordance with a conventional method at 130°C for 60 minutes.

Evaluation of Physical Properties after Crosslinking:

A sheet after the crosslinking was cut as appropriate for evaluation items to provide test pieces. The following physical property evaluations were performed using the test pieces.

Tensile Test:

Under an original state and after thermal aging (at 100°C for 22 hours), a tensile test was performed by a method in conformity to JIS-K 6301. In this test, moduli at 300% and 500% elongation, tensile strength, break elongation, and surface hardness (JIS-type A) at room temperature were measured.

Deformation Ratio:

At room temperature, a test piece of a strip shape having a width of 6 mm and a length of 100 mm was cut out of the crosslinked film, and the test piece was pulled to an elongation of 300% at a gauge length of 10 mm, kept in this state for 10 minutes, and then released. After 10 minutes, an elongation in gauge length was measured, and a deformation ratio was calculated as the ratio of displacement from the initial position of a gauge mark.

Temporal Stability of Flexibility:

As an accelerated test, the film after the crosslinking was evaluated for its modulus of elasticity after storage at each of low temperature (-10°C for 50 days) and high temperature (70°C for 7 days).

Production Example: Preparation of Chloroprene-based Polymer Latex

A reaction vessel having an internal volume of 60 L was used and fed with 18.2 kg of 2-chloro-1,3-butadiene (chloroprene), 1.8 kg of 2,3-dichloro-1,3-butadiene, 18 kg of pure water, 860 g of disproportionated rosin acid (R-300 manufactured by Arakawa Chemical Industries, Ltd.), 2.0 g of n-dodecyl mercaptan, 240 g of potassium hydroxide, and 160 g of a sodium salt of a β-naphthalenesulfonic acid-formaldehyde condensate. The contents were emulsified to convert the disproportionated rosin acid into a rosin soap, and then polymerization was performed using potassium persulfate as an initiator under a nitrogen atmosphere at 40°C. As soon as the polymerization conversion reached 88.1%, an emulsion of phenothiazine was added to terminate the polymerization. Then, unreacted monomers were removed by steam distillation. Thus, a chloroprene polymer-based polymer latex was obtained.

Example 1:

A compound was prepared with the chloroprene-based polymer latex obtained by the method of the above-mentioned production example in the compounding ratio shown in Table 1, and a dipped and crosslinked film was produced.

Examples 2 to 5, and Comparative Examples 1 and 2:

Chloroprene-based polymer latexes to be used in Examples 2 to 5, and Comparative Examples 1 and 2 were each obtained by performing polymerization in the same manner as in the above-mentioned production example except that the amount of 2,3-dichloro-1,3-butadiene, the amount of n-dodecyl mercaptan, and the polymerization conversion were changed. Compounds were prepared with the obtained chloroprene-based polymer latexes in the compounding ratio shown in Table 1, and dipped and crosslinked films were produced.

Comparative Examples 3 to 5:

Chloroprene-based polymer latexes to be used in Comparative Examples 3 to 5 were each obtained by performing polymerization in the same manner as in the above-mentioned production example except that the amount of 2,3-dichloro-1,3-butadiene, the amount of n-dodecyl mercaptan, and the polymerization conversion were changed. Compounds were prepared with the obtained chloroprene-based polymer latexes in the compounding ratio shown in Table 2 below, and dipped and crosslinked films were produced.

Table 2

|  | Part(s) by mass |
| --- | --- |
| Chloroprene-based polymer latex | 100 |
| Surfactant (1) | 1 |
| Surfactant (2) | 2 |
| Zinc oxide dispersion (3) | 5 |
| Phenol-based antioxidant dispersion (4) | 2 |
| Accelerator TP aqueous solution (5) | 1 |
| Accelerator TETD dispersion (6) | 1 |
| Glycine | 0.5 |

Notes:
(1) Darvan SMO manufactured by Kawaguchi Chemical Industry, Co., Ltd.
(2) Darvan WAQ manufactured by Kawaguchi Chemical Industry, Co., Ltd.
(3) AZ-SW manufactured by Osaki Industry, Co., Ltd.
(4) K-840 (Wingstay (trademark) L dispersion) manufactured by Chukyo Yushi Co., Ltd.
(5) NOCCELER TP (sodium dibuthyldithiocarbamate) manufactured by Ouchi Shinko Chemical Industrial Co., Ltd.
(6) NOCCELER TET (tetraethylthiuram disulfide) manufactured by Ouchi Shinko Chemical Industrial Co., Ltd.
(6) was prepared as an aqueous dispersion in advance of its addition.

Table 3 collectively shows the results of Examples 1 to 5 and Comparative Examples 1 to 5 above.

Table 3

| | | Example 1 | Example 2 | Example 3 | Example 4 | Example 5 | Comparative Example 1 | Comparative Example 2 | Comparative Example 3 | Comparative Example 4 | Comparative Example 5 |
|---|---|---|---|---|---|---|---|---|---|---|---|
| Polymerization condition | Polymerization temperature | 40 | 40 | 30 | 35 | 35 | 40 | 40 | 40 | 35 | 40 |
| | Use amount of 2,3-dichlorobutadiene (mass%) | 10 | 10 | 13.5 | 15 | 11.5 | 8.5 | 10 | 10 | 13.5 | 8.5 |
| | n-Dodecyl mercaptan (mass%) | 0.001 | 0.020 | 0.010 | 0.015 | 0.015 | 0.060 | 0.050 | 0.001 | 0.060 | 0.060 |
| | Polymerization conversion (%) | 89.1 | 84.9 | 88.1 | 90.3 | 89.2 | 86.8 | 83.3 | 82.1 | 85.4 | 86.8 |
| Compounding ratio of composition | Chloroprene-based polymer latex | 100 | 100 | 100 | 100 | 100 | 100 | 100 | 100 | 100 | 100 |
| | Surfactant (1) | 1 | 1 | 1 | 1 | 1 | 1 | 1 | 1 | 1 | 1 |
| | Surfactant (2) | 2 | 2 | 2 | 2 | 2 | 2 | 2 | 2 | 2 | 2 |
| | Zinc oxide dispersion 3) | 5 | 5 | 5 | 5 | 5 | 5 | 5 | 5 | 5 | 5 |
| | Phenol-based antioxidant (4) | 2 | 2 | 2 | 2 | 2 | 2 | 2 | 2 | 2 | 2 |
| | Accelerator TP dispersion (5) | - | - | - | - | - | - | - | 1 | 1 | 1 |
| | Accelerator TETD dispersion (6) | - | - | - | - | - | - | - | 1 | 1 | 1 |
| | Glycine | 0.5 | 0.5 | 0.5 | 0.5 | 0.5 | 0.5 | 0.5 | 0.5 | 0.5 | 0.5 |

(continued)

| | Example No. | Example 1 | Example 2 | Example 3 | Example 4 | Example 5 | Comparative Example 1 | Comparative Example 2 | Comparative Example 3 | Comparative Example 4 | Comparative Example 5 |
|---|---|---|---|---|---|---|---|---|---|---|---|
| Physical property test results | Tetrahydrofuran insoluble fraction (%) | 83 | 55 | 77 | 62 | 69 | 36 | 29 | 82 | 28 | 36 |
| | Copolymerization fraction of 2,3-dichlorobutadiene (%) | 11.2 | 11.8 | 15.2 | 16.0 | 12.8 | 9.5 | 12.0 | 10.2 | 12.6 | 9.5 |
| | Tensile properties (vulcanization at 130°C for 60 minutes) Deformation ratio (%) | 10 | 10 | 10 | 10 | 8 | 13 | 14 | 6 | 9 | 6 |
| | Modulus at 300% elongation (MPa) | 0.9 | 0.8 | 0.8 | 0.9 | 0.9 | 0.7 | 0.5 | 1.3 | 1.0 | 1.0 |
| | Modulus at 500% elongation (MPa) | 1.7 | 1.5 | 1.6 | 1.6 | 1.6 | 1.0 | 0.9 | 1.8 | 1.2 | 1.4 |
| | Tensile strength (MPa) | 21.2 | 19.9 | 17.6 | 17.1 | 20.4 | 16.4 | 15.5 | 28.6 | 18.6 | 24.1 |
| | Elongation at break (%) | 1,150 | 1,200 | 1,210 | 1,200 | 1,180 | 1,300 | 1,330 | 1,000 | 1,180 | 950 |

(continued)

| Example No. | Example 1 | Example 2 | Example 3 | Example 4 | Example 5 | Comparative Example 1 | Comparative Example 2 | Comparative Example 3 | Comparative Example 4 | Comparative Example 5 |
|---|---|---|---|---|---|---|---|---|---|---|
| Temporal stability of flexibility (vulcanization at 130°C for 60 minutes)<br>After 7 days at 70°C<br>Modulus at 300% elongation (MPa) | 1.0 | 1.0 | 0.9 | 1.1 | 1.1 | 0.7 | 0.6 | 1.8 | 2.1 | 2.0 |
| Modulus at 500% elongation (MPa)<br>After 50 days at -10°C<br>Modulus at 300% elongation (MPa) | 1.8<br><br><br>1.2 | 1.5<br><br><br>1.0 | 1.5<br><br><br>1.4 | 1.6<br><br><br>1.3 | 1.6<br><br><br>1.4 | 0.9<br><br><br>0.8 | 1.0<br><br><br>0.8 | 2.5<br><br><br>1.7 | 2.8<br><br><br>1.5 | 2.8<br><br><br>1.5 |
| Modulus at 500% elongation (MPa) | 2.2 | 1.8 | 1.8 | 1.7 | 2.0 | 1.0 | 1.2 | 2.3 | 2.8 | 2.6 |

Notes: (1) Darvan SMO manufactured by Kawaguchi Chemical Industry Co., Ltd.
(2) Darvan WAQ manufactured by Kawaguchi Chemical Industry Co., Ltd.
(3) AZ-SW manufactured by Osaki Industry Co., Ltd.
(4) K-840 (Wingstay (trademark) L dispersion) manufactured by Chukyo Yushi Co., Ltd.
(5) NOCCELER TP (sodium dibutyldithiocarbamate) manufactured by Ouchi Shinko Chemical Industrial Co., Ltd.
(6) NOCCELER TET (tetraethylthiuram disulfide) manufactured by Ouchi Shinko Chemical Industrial Co., Ltd.

(6) was prepared as an aqueous dispersion in advance of its addition.

In the case where the composition of the invention of the present application is molded into a glove, when the numerical value for the modulus at 300% elongation is high, a returning force against a finger being bent is strong and the sense of use is hard. In addition, when the numerical value for the modulus at 300% elongation is low, the sense of use is soft and even long-term use hardly causes fatigue. Therefore, it is found from the results shown in Table 3 that the glove obtained in the compounding ratio of Comparative Example 3 has a hard sense of use.

It is also found from the results shown in Table 3 that the tensile strength of the molded article obtained in the compounding ratio of each of Comparative Examples 1 and 2 is insufficient as a surgical glove.

**Claims**

1.   A composition for rubber, comprising a chloroprene-based polymer latex (A), a metal oxide (B), an antioxidant (C), a surfactant (D) and a pH adjuster (E), and being free of a vulcanization accelerator, in which a tetrahydrofuran insoluble matter content in a chloroprene-based polymer in the (A) is from 50 mass% to 85 mass%, and in which contents of the (B), the (C), the (D), and the (E) with respect to 100 parts by mass of a solid content of the (A) are from 1 part by mass to 10 parts by mass, from 0.1 part by mass to 5 parts by mass, from 0.1 part by mass to 10 parts by mass, and from 0.01 part by mass to 5 parts by mass, respectively.

2.   The composition for rubber according to claim 1, which is a composition consisting of the chloroprene-based polymer latex (A), the metal oxide (B), the antioxidant (C), the surfactant (D), and the pH adjuster (E).

3.   The composition for rubber according to claim 1 or 2, in which a polymer contained in the chloroprene-based polymer latex (A) is a copolymer formed from monomers comprising 2-chloro-1,3-butadiene (chloroprene) (A-1) and 2,3-dichloro-1,3-butadiene (A-2), and in which ratios of the monomers with respect to 100 mass% in total of the 2-chloro-1,3-butadiene (chloroprene) (A-1) and the 2,3-dichloro-1,3-butadiene (A-2) are 76 mass% to 93 mass% of the 2-chloro-1,3-butadiene (chloroprene) (A-1) and 24 mass% to 7 mass% of the 2,3-dichloro-1,3-butadiene (A-2).

4.   The composition for rubber according to claim 3, in which the polymer contained in the chloroprene-based polymer latex (A) is a copolymer formed from the 2-chloro-1,3-butadiene (chloroprene) (A-1) and the 2,3-dichloro-1,3-butadiene (A-2), and further, a monomer (A-3) copolymerizable therewith, and in which a content of the monomer (A-3) is from 0.1 part by mass to 10 parts by mass with respect to 100 parts by mass in total of the 2-chloro-1,3-butadiene (chloroprene) (A-1) and the 2,3-dichloro-1,3-butadiene (A-2).

5.   A molded article, which is obtained using the composition for rubber according to any one of claims 1 to 4.

6.   The molded article according to claim 5, which has a 300% modulus of elasticity of from 0.5 MPa to 1.2 MPa, a tensile strength of 17 MPa or more, a tensile elongation at break of 800% or more, and a deformation ratio of 20% or less.

7.   A dipped rubber article, which is obtained using the composition for rubber according to any one of claims 1 to 4.

8.   The dipped rubber article according to claim 7, in which the dipped rubber article is a glove.

9.   The dipped rubber article according to claim 8, in which the glove is a disposable medical glove.

**Patentansprüche**

1.   Zusammensetzung für Kautschuk, umfassend einen Polymerlatex auf Chloroprenbasis (A), ein Metalloxid (B), ein Antioxidationsmittel (C), ein Tensid (D) und ein pH-Stellmittel (E), und die frei von einem Vulkanisationsbeschleuniger ist, wobei der Gehalt an Tetrahydrofuran-unlöslichen Stoffen in einem Polymer auf Chloroprenbasis in der Komponente (A) von 50 Massen-% bis 85 Massen-% beträgt, und wobei die Gehalte der Komponente (B), der Komponente (C), der Komponente (D) und der Komponente (E), jeweils bezogen auf 100 Massenteile eines Feststoffgehalts von (A), von 1 Massenteil bis 10 Massenteile, von 0,1 Massenteil bis 5 Masseteile, von 0,1 Massenteil bis 10 Massenteile und von 0,01 Massenteil bis 5 Massenteile betragen.

2.   Zusammensetzung für Kautschuk nach Anspruch 1, bei der es sich um eine Zusammensetzung handelt, die aus dem Polymerlatex auf Chloroprenbasis (A), dem Metalloxid (B), dem Antioxidationsmittel (C), dem Tensid (D) und dem pH-Stellmittel (E) besteht.

3. Zusammensetzung für Kautschuk nach Anspruch 1 oder 2, bei der ein in dem Polymerlatex auf Chloroprenbasis (A) enthaltenes Polymer ein Copolymer ist, das aus Monomeren gebildet ist, die 2-Chlor-1,3-butadien (Chloropren) (A-1) und 2,3-Dichlor-1,3-butadien (A-2) umfassen, und wobei die Verhältnisse der Monomere, bezogen auf 100 Massen-% insgesamt des 2-Chlor-1,3-Butadiens (Chloropren) (A-1) und des 2,3-Dichlor-1,3-Butadiens (A-2), 76 Massen-% bis 93 Massen-% des 2-Chlor-1,3-Butadiens (Chloropren) (A-1) und 24 Massen-% bis 7 Massen-% des 2,3-Dichlor-1,3-Butadiens (A-2) betragen.

4. Zusammensetzung für Kautschuk nach Anspruch 3, in der das in dem Polymerlatex auf Chloroprenbasis (A) enthaltene Polymer ein Copolymer ist, das aus dem 2-Chlor-1,3-butadien (Chloropren) (A-1) und dem 2,3-Dichlor-1,3-butadien (A-2) und weiterhin einem damit copolymerisierbaren Monomer (A-3) gebildet ist, wobei der Gehalt des Monomers (A-3) von 0,1 Massenteil bis 10 Massenteile in Bezug auf insgesamt 100 Massenteile des 2-Chlor-1,3-Butadiens (Chloropren) (A-1) und des 2,3-Dichlor-1,3-Butadiens (A-2) beträgt.

5. Formkörper, der unter Verwendung der Zusammensetzung für Kautschuk nach einem der Ansprüche 1 bis 4 erhalten wird.

6. Formkörper nach Anspruch 5, der einen 300%-Elastizitätsmodul von 0,5 MPa bis 1,2 MPa, eine Zugfestigkeit von 17 MPa oder mehr, eine Zugbruchdehnung von 800% oder mehr und ein Verformungsverhältnis von 20% oder weniger aufweist.

7. Tauchkautschuk (dipped rubber)-Gegenstand, der unter Verwendung der Zusammensetzung für Kautschuk nach einem der Ansprüche 1 bis 4 erhalten wird.

8. Tauchkautschuk (dipped rubber)-Gegenstand nach Anspruch 7, wobei der Tauchkautschuk-Gegenstand ein Handschuh ist.

9. Tauchkautschuk (dipped rubber)-Gegenstand nach Anspruch 8, wobei der Handschuh ein medizinischer Einmalhandschuh ist.

**Revendications**

1. Composition pour caoutchouc, comprenant un latex de polymère à base de chloroprène (A), un oxyde métallique (B), un antioxydant (C), un agent tensioactif (D) et un ajusteur de pH (E), et étant exempte d'accélérateur de vulcanisation, dans laquelle une teneur en matières insolubles dans le tétrahydrofurane dans un polymère à base de chloroprène dans le (A) est de 50 % en masse à 85 % en masse, et dans laquelle les teneurs du (B), du (C), du (D) et du (E) par rapport à 100 parties en masse d'une teneur en matières solides du (A) sont de 1 partie en masse à 10 parties en masse, de 0,1 partie en masse à 5 parties en masse, de 0,1 partie en masse à 10 parties en masse, et de 0,01 partie en masse à 5 parties en masse, respectivement.

2. Composition pour caoutchouc selon la revendication 1, qui est une composition constituée du latex de polymère à base de chloroprène (A), de l'oxyde métallique (B), de l'antioxydant (C), de l'agent tensioactif (D) et de l'ajusteur de pH (E).

3. Composition pour caoutchouc selon la revendication 1 ou 2, dans laquelle un polymère contenu dans le latex de polymère à base de chloroprène (A) est un copolymère formé à partir de monomères comprenant du 2-chloro-l,3-butadiène (chloroprène) (A-1) et du 2,3-dichloro-1,3-butadiène (A-2), et dans laquelle les rapports des monomères par rapport à 100 % en masse au total du 2-chloro-1,3-butadiène (chloroprène) (A-1) et du 2,3-dichloro-1,3-butadiène (A-2) sont de 76 % en masse à 93 % en masse du 2-chloro-1,3-butadiène (chloroprène) (A-1) et de 24 % en masse à 7 % en masse du 2,3-dichloro-1,3-butadiène (A-2).

4. Composition pour caoutchouc selon la revendication 3, dans laquelle le polymère contenu dans le latex de polymère à base de chloroprène (A) est un copolymère formé à partir du 2-chloro-1,3-butadiène (chloroprène) (A-1) et du 2,3-dichloro-1,3-butadiène (A-2), et en outre, d'un monomère (A-3) copolymérisable avec ceux-ci, et dans laquelle une teneur du monomère (A-3) est de 0,1 partie en masse à 10 parties en masse par rapport à 100 parties en masse au total du 2-chloro-1,3-butadiène (chloroprène) (A-1) et du 2,3-dichloro-1,3-butadiène (A-2).

**5.** Article moulé, qui est obtenu en utilisant la composition pour caoutchouc selon l'une quelconque des revendications 1 à 4.

**6.** Article moulé selon la revendication 5, qui a un module d'élasticité à 300 % de 0,5 MPa à 1,2 MPa, une résistance à la traction de 17 MPa ou plus, un allongement à la traction à la rupture de 800 % ou plus, et un rapport de déformation de 20 % ou moins.

**7.** Article en caoutchouc d'immersion, qui est obtenu en utilisant la composition pour caoutchouc selon l'une quelconque des revendications 1 à 4.

**8.** Article en caoutchouc d'immersion selon la revendication 7, dans lequel l'article en caoutchouc d'immersion est un gant.

**9.** Article en caoutchouc d'immersion selon la revendication 8, dans lequel le gant est un gant médical jetable.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007106994 A
- JP 2009501833 A

- EP 1904569 B1